Europäisches Patentamt

European Patent Office

Office européen des brevets

(19)

(11) Publication number: **0 277 261**
**A1**

(12) **EUROPEAN PATENT APPLICATION**

(21) Application number: **87104287.5**

(22) Date of filing: **24.03.87**

(51) Int. Cl.⁴: **A61F 5/01**

(30) Priority: **26.01.87 IL 81390**

(43) Date of publication of application:
**10.08.88 Bulletin 88/32**

(84) Designated Contracting States:
**AT BE CH DE ES FR GB GR IT LI LU NL SE**

(71) Applicant: **INTEC Advanced Computerized Systems Medical Ltd.**
**Hillel Street, 5**
**Jerusalem(IL)**

(72) Inventor: **Livni, Michael, Dr.**
**6/1 Hazav St.**
**Mevasseret(IL)**

(74) Representative: **Dreiss, Hosenthien & Fuhlendorf**
**Gerokstrasse 6**
**D-7000 Stuttgart 1(DE)**

(54) **Orthopedic diaper.**

(57) A diaper suitable for the preventive treatment of a congenital defect, congenital dislocation or dysplasia of the hip joint for infants comprises means integral with the same or appropriately insertable into the same. Said means being non-water absorbent and being located, in the set-up position of the diaper, at the groin, between the thighs and supporting them in an abducted position.

## FIG. 1

The present invention is directed to a diaper suitable for the preventive treatment of a congenital defect, congenital dislocation or dysplasia of the hip joint also in cases not diagnosed as such, immediately or shortly after birth. There is some confusion in the use of these terms, since dysplasia means only a dislocatable hip joint, and a distinction is important when considering the incidence of the defect. On the average, according to some authorities, the incidence of dysplasia seems to be about one percent of live births, but the fully developed cases of dislocated hips are considerably less frequent, also if not treated. This leads some authorities to the conclusion that there is a possibility of spontaneous stabilization of dysplastic hips in early infancy.

In any case, where there is only mild dysplasia it might not always be diagnosed even by trained personnel, especially during earliest infancy. Therefore it is possible that a number of cases may go undetected until the child is two or three months old, when X-ray examination will provide conclusive proof of whether there is a dysplasia/dislocation or not. The presence of such abnormalities calls for treatment by immobilization with appropriate splints, usually keeping the legs abducted at approximately 60° and appropriately flexed. This posture which allows the head of the femur to exert pressure on the acetabulum (socket) has been proven to enhance good development as well as stabilization of the hip joint.

An adducted position of the thighs in infancy may lead to a higher indicence of dysplastic, respectively dislocated hips. This is supported by observations of such a higher incidence in populations where it is customary to swaddle infants with thighs in an adducted position. The importance of holding the thighs in an abducted position is now well recognised, and the present widespread practice is to use a makeshift arrangement, i.e., to use two or perhaps more layers of diapers, providing some semi-rigidity and also a kind of wad in the region of the groin to achieve abduction. However, this practice is only partially effective as the material of the diapers is water absorbent and loses almost all rigidity and resistance on becoming wet. Furthermore, it involves additional work, expense, and inconvenience.

As to the splints used in the therapeutical treatment of diagnosed cases (not forming part of the present invention), these are in most cases very rigid splints. However, the use of foamed plastic cushions, trapped to the thighs and permitting certain freedom of adductive movements but capable of recovering their shape, has also been recommended in the literature.

In view of the fact that the present invention contemplates only non-water absorbent means for holding the thighs of the infant in the abducted position, the problem of becoming ineffective in the wet state does not arise, and this constitutes a considerable advantage of the present invention over the prior art makeshift or solutions.

Therefore the present invention provides a diaper for infants characterised in that it comprises means integral with the same or appropriately insertable into the same, said means being non-water absorbent and being located in the set-up position of the diaper, at the groin, between the thighs and supporting them in an abducted position. Several embodiments are suitable for realizing the present invention.

It seems to be desirable to avoid use of relatively hard and overly rigid materials, which may bruise the delicate skin of the infant. Therefore inflated cushions and foamed plastic cushions for holding the thighs in the desired position appear preferable. Plastic strips having suitable stiffness and elasticity and suitably cushioned are also contemplated.

Since most modern mothers are disinclined to launder diapers, the embodiment involving disposable diapers will be most common at present, but the invention may be equally well applied to reusable fabric diapers which may be more economical in some cases. Of course, in case of reusable diapers, the embodiment involving separate insertible means is preferable, as diapers integrally incorporating said means may be inconvenient to launder.

In the case of using separate insertibel means, these are held in appropriate pockets, integrally formed with the diaper proper.

The present invention is described in more detail on hand of the accompanying drawings.

-Fig. 1 represents a general view of an infant wearing a diaper according to the invention;

-Fig. 2 represents an embodiment of the invention using plastic strips;

-Fig. 3 represents an embodiment with a cushion inserted in a pocket;

-Fig. 4 represents an embodiment using an inflatable cushion;

-Fig. 4a represents a side view of the embodiment of Fig. 4;

-Fig. 4b represents the embodiment of Figs. 4 and 4a in an exploded side view; and

-Fig. 4c represents a modification of the embodiment of Figs. 4, 4a and 4b.

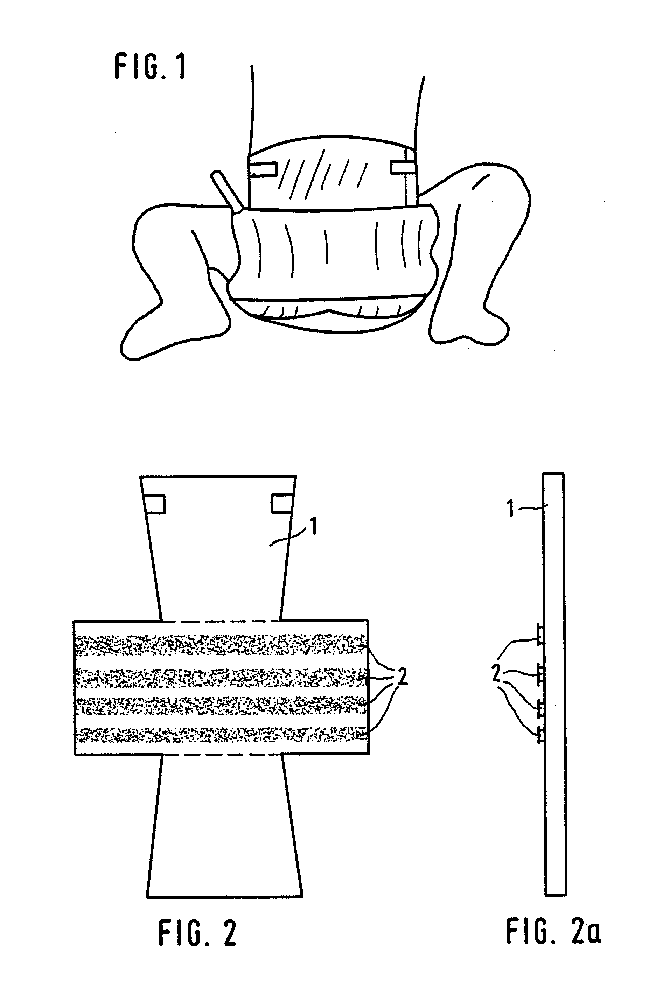

Fig. 1 is a schematic general view, mainly to visualise the position the infant's thighs assume when wearing the diaper of the present invention, the means supporting the thighs are only indicated by an arrow in a general manner.

In Fig. 2 plastic strips 2 are affixed on the side of the non-absorbent layer 1 of the diaper which faces away from the infant's body. As illustrated in the drawing, said plastic strips are embossed, but this is not an absolute requirement. The object of the embossing is only to impart suitable elasticity/rigidity characteristics, but this object may also be accomplished by selecting strips of appropriate material, width and thickness. In the drawing the strips are simply affixed to the outermost layer of the diaper, which may be done by a suitable adhesive, or possibly by pressing them on, but it is also possible to hold the strips in place by an additional outer layer, which may be water impermeable if desired. This possibility is not illustrated in the drawing but is self-evident to the expert.

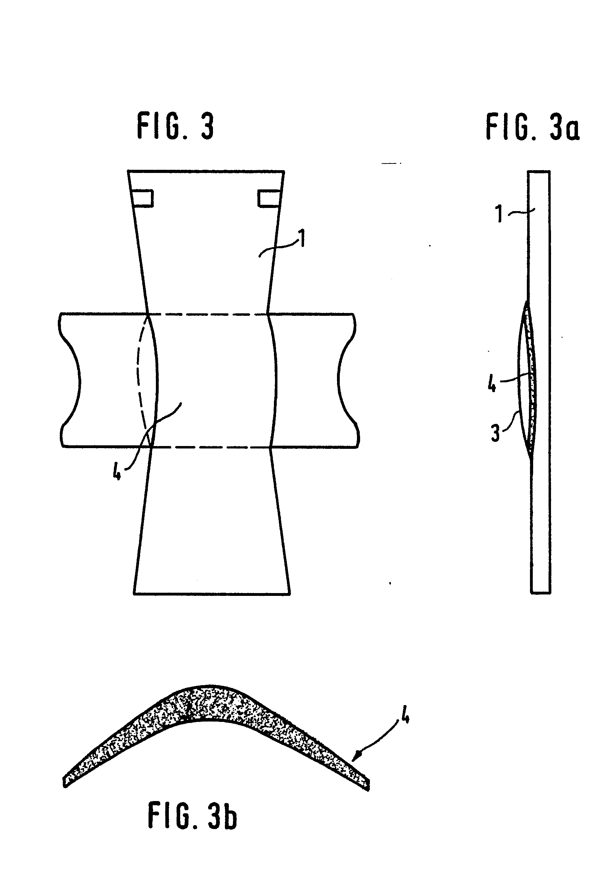

The embodiment illustrated in Fig. 3 may be used either with a disposable or with a reusable fabric diaper. A pocket 3 is formed on the side of diaper 1 facing away from the infant's body, said pocket may be open at both sides or only at one side and is adapted for insertion thereinto of a cushion 4 having adequate elasticity and stiffness, the concave face of said cushion facing away from the infant's body. This cushion may be, as an example, of a suitable foamed plastic material, reusable or disposable. Of course an inflatable cushion may be also inserted into said pocket, this possibility is not illustrated.

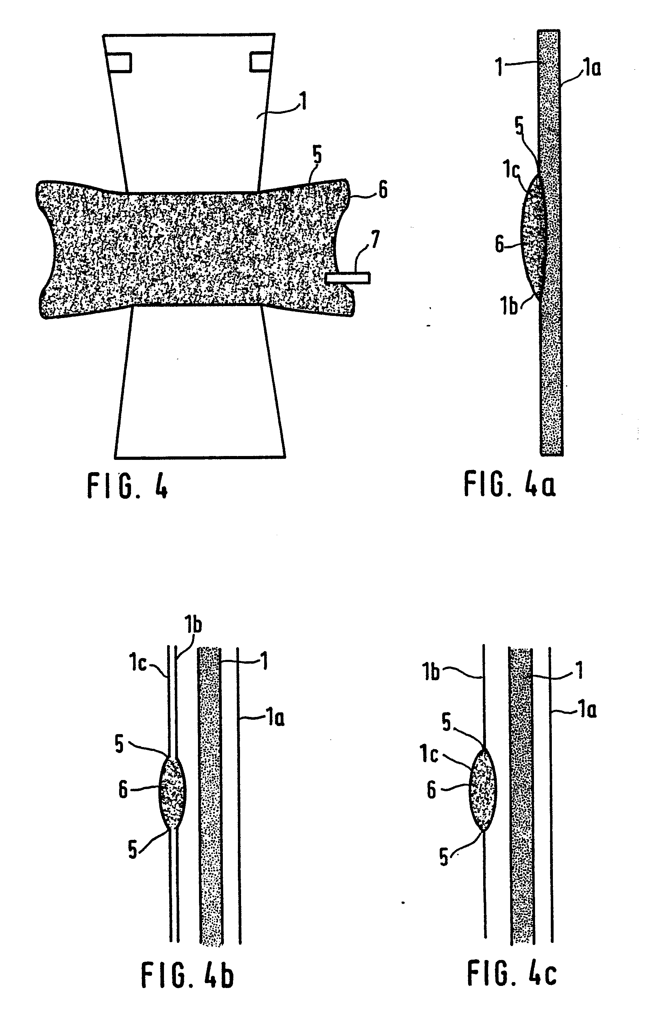

The embodiment illustrated in Figs. 4, 4a and 4b is possibly the most preferred one and is illustrated in more detail. In the side view (4a) and in the exploded view (4b), the numeral 1 designates the absorbing layer of the diaper, 1a is a permeable tissue layer facing the body of the infant, 1b and 1c designate two heat sealable plastic layers on the outermost side, these two layers are in close contact over the entire face of the diaper, except in the area deliminated by heat sealing line 5. As the two layers are air tight sealed together along this line, leaving a suitable free opening for inflating an inflatable cushion 6 is formed between them in the area deliminated by said heat sealing line. A short length of tubing 7 containing a self-sealing valve may be inserted in said opening, or also a tubing may be formed by suitably extending said heat sealable layers beyond the absorbing layer and appropriately configuring the heat sealing line.

In the modification illustrated in Fig. 4c there is only one layer 1b of plastic sheet covering most of the surface of the diaper and a second layer 1c is heat sealed to this layer only in the region forming said inflatable cushion. The inflating may be done by mouth or by a small aerosol containing a suitable gas. Also the possibility of inflating by a gas-producing mixture of chemicals.

While several specific embodiments have been described hereinabove in details, the invention is not limited thereto and is only defined by the scope of the appended claims.

## Claims

1. A diaper for infants characterised in that it comprises means integral with the same or appropriately insertable into the same, said means being non-water absorbent and being located, in the set-up position of the diaper, at the groin, between the thighs and supporting them in an abducted position.

2. A diaper according to claim 1 wherein said diaper is a disposable diaper.

3. A diaper according to claim 1 or claim 2 wherein said means form an integral part of the diaper.

4. A diaper according to claim 3 wherein said means comprise an inflatable cushion formed on the face of the diaper away from the infant's body.

5. A diaper according to claim 4 wherein the diaper comprises two layers of water-impermeable heat-sealable plastic sheets on the side facing away from the infant's body and said inflatable cushion is formed by heat-sealing said sheets together along a line in the region between the thighs leaving a closable opening for inflating said cushion.

6. A diaper according to claim 4 wherein the diaper comprises one layer of water impermeable, heat sealable plastic sheet on the side facing away from the infant's body, a second layer of water impermeable, heat sealable plastic sheet being heat sealed to said plastic sheet in the region of said inflatable cushion between the thighs, leaving a closable opening for inflating said cushion.

7. A diaper according to claim 3 wherein said means are formed by one or a plurality of plastic strips which may be embossed, having an appropriate elasticity, affixed or held in place by an additional outer layer in the region between the thighs to or on the side of the diaper facing away from the infant's body.

8. A diaper according to claim 1 wherein said diaper is a disposable diaper or a reusable fabric diaper having an open pocket in the region between the thighs, said pocket being adapted for insertion thereinto of means, said means being disposable or reusable, supporting the thighs, in an abducted position.

9. A diaper according to claim 8, wherein said pocket is formed by a sleeve, open at both ends thereof.

10. A diaper according to claim 8 wherein said means are formed by a cushion of non-water absorbent material having adequate stiffness and elasticity.

11. A diaper according to claim 10 wherein said cushion is of a formed plastics material.

12. A diaper according to claim 8 wherein said means are formed by a plastics strip having appropriate elasticity which strip may be embossed.

13. A diaper according to claim 8 wherein said means are formed by an inflatable cushion of non-water absorbent material.

FIG. 1

FIG. 2

FIG. 2a

FIG. 3

FIG. 3a

FIG. 3b

FIG. 4

FIG. 4a

FIG. 4b

FIG. 4c

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 050 050 (LAMBERT) <br> * claims 1, 2; figures 1-7 * | 1 | A 61 F 5/01 |
| A | FR-A-2 050 637 (W. JUDICKE KG) <br> * claims 1, 2 * | 1 | |
| A | DE-A-3 316 903 (BABY-CHIC FINSTERWALDE) <br> * claims 1, 3; figures 1-3 * | 1 | |
| A | DE-A-1 491 199 (NICOLL) <br> * page 25; lines 1-17 * | 4,5 | |

TECHNICAL FIELDS SEARCHED (Int. Cl.4)

A 61 F 5/00

The present search report has been drawn up for all claims

| Place of search | Date of completion of the search | Examiner |
|---|---|---|
| BERLIN | 12-04-1988 | KANAL P K |